# EUROPEAN PATENT APPLICATION

(11) **EP 3 915 561 A1**
(43) Date of publication of application: **01.12.2021**
(21) Application number: 21171525.5
(22) Date of filing: 11.09.2014
(51) Int. Cl.: A61K 31/4545, A61P 25/06, A61K 47/10, A61K 9/08, A61P 43/00

(54) **FORMULATIONS FOR CGRP RECEPTOR ANTAGONISTS**

(30) Priority: 16.09.2013 US 201361878183 P
(62) Divisional of application: 14844555.4
(71) Applicant: MERCK SHARP & DOHME CORP., Rahway, NJ 07065-0907 (US)
(72) Inventor: Mahjour, Majid, West Point, Pennsylvania, 19486 (US); Allain, Leonardo R., West Point, Pennsylvania, 19486 (US); Sotthivirat, Sutthilug, West Point, Pennsylvania, 19486 (US); Maus, Russell G., West Point, Pennsylvania, 19486 (US); Nofsinger, Rebecca, West Point, Pennsylvania, 19486 (US); Lupton, Lisa, Foster City, California, 94404 (US); Xu, Wei, West Point, Pennsylvania, 19486 (US); Flanagan, Francis, West Point, Pennsylvania, 19486 (US)
(74) Representative: Hoffmann Eitle

(57) **Abstract**

The instant invention relates to liquid pharmaceutical compositions containing CGRP receptor antagonists. The CGRP receptor antagonist liquid pharmaceutical compositions of the instant invention are alcohol-free, low volume liquid pharmaceutical compositions that can be taken without water for the treatment of migraine headache.

## Description

### BACKGROUND OF THE INVENTION

This invention relates to liquid pharmaceutical compositions of CGRP Receptor antagonists.

CGRP is a potent neuromodulator that has been implicated in the pathology of cerebrovascular disorders such as migraine and cluster headache. In clinical studies, elevated levels of CGRP in the jugular vein were found to occur during migraine attacks (Goadsby et al. (1990) Ann. Neurol. 28, 183-187), salivary levels of CGRP are elevated in migraine subjects between (Bellamy et al. (2006) Headache 46, 24-33) and during attacks (Cady et al. (2009) Headache 49, 1258-1266), and CGRP itself has been shown to trigger migrainous headache (Lassen et al. (2002) Cephalalgia 22, 54-61). In clinical trials, the CGRP receptor antagonist BIBN4096BS has been shown to be effective in treating acute attacks of migraine (Olesen et al. (2004) New Engl. J. Med. 350, 1104-1110) and was able to prevent headache induced by CGRP infusion in a control group (Petersen et al. (2005) Clin. Pharmacol. Ther. 77, 202-213). The orally bioavailable CGRP receptor antagonist telcagepant has also shown antimigraine effectiveness in phase III clinical trials (Ho et al. (2008) Lancet 372, 2115-2123; Connor et al. (2009) Neurology 73, 970-977).

CGRP receptor antagonists may be useful pharmacological agents for disorders that involve CGRP in humans and animals, but particularly in humans. Such disorders include migraine and cluster headache (Doods (2001) Curr. Opin. Invest. Drugs 2, 1261-1268; Edvinsson et al. (1994) Cephalalgia 14, 320-327); chronic tension type headache (Ashina et al. (2000) Neurology 14, 1335-1340); pain (Yu et al. (1998) Eur. J. Pharmacol. 347, 275-282); chronic pain (Hulsebosch et al. (2000) Pain 86, 163-175); neurogenic inflammation and inflammatory pain (Holzer (1988) Neuroscience 24, 739-768; Delay-Goyet et al. (1992) Acta Physiol. Scanda. 146, 537-538; Salmon et al. (2001) Nature Neurosci. 4, 357-358); eye pain (May et al. (2002) Cephalalgia 22, 195-196), tooth pain (Awawdeh et al. (2002) Int. Endocrin. J. 35, 30-36), non-insulin dependent diabetes mellitus (Molina et al. (1990) Diabetes 39, 260-265); vascular disorders; inflammation (Zhang et al. (2001) Pain 89, 265); arthritis, bronchial hyperreactivity, asthma, (Foster et al. (1992) Ann. NY Acad. Sci. 657, 397-404; Schini et al. (1994) Am. J. Physiol. 267, H2483-H2490; Zheng et al. (1993) J. Virol. 67, 5786-5791); shock, sepsis (Beer et al. (2002) Crit. Care Med. 30, 1794-1798); opiate withdrawal syndrome (Salmon et al. (2001) Nature Neurosci. 4, 357-358); morphine tolerance (Menard et al. (1996) J. Neurosci. 16, 2342-2351); hot flashes in men and women (Chen et al. (1993) Lancet 342, 49; Spetz et al. (2001) J. Urology 166, 1720-1723); allergic dermatitis (Wallengren (2000) Contact Dermatitis 43, 137-143); psoriasis; encephalitis, brain trauma, ischaemia, stroke, epilepsy, and neurodegenerative diseases (Rohrenbeck et al. (1999) Neurobiol. Dis. 6, 15-34); skin diseases (Geppetti and Holzer, Eds., Neurogenic Inflammation, 1996, CRC Press, Boca Raton, FL), neurogenic cutaneous redness, skin rosaceousness and erythema; tinnitus (Herzog et al. (2002) J. Membr. Biol. 189, 225); obesity (Walker et al. (2010) Endocrinology 151, 4257-4269); inflammatory bowel disease, irritable bowel syndrome, (Hoffman et al. (2002) Scand. J. Gastroenterol. 37, 414-422) and cystitis. Of particular importance is the acute or prophylactic treatment of headache, including migraine and cluster headache.

Representative examples of CGRP receptor antagonists include those disclosed in International Publication WO2012/064910, which published on May 18, 2012, to Merck Sharp & Dohme Corp., which is hereby incorporated by reference in its entirety.

CGRP receptor antagonists can be formulated for oral dosing as tablets, by using a various methods, including hot melt extrusion and spray drying. Similarly, CGRP receptor antagonists can be formulated for oral dosing as gelatin capsules, as a liquid in a soft capsule, or dry powder or semi-solid in a hard capsule. In addition, CGRP receptor antagonists can be formulated for intravenous dosing.

The CGRP receptor antagonist liquid pharmaceutical compositions of the instant invention are alcohol-free, low volume liquid pharmaceutical compositions that can be taken without water for the treatment of migraine headache. The liquid pharmaceutical compositions of the instant invention have advantages over other liquid compositions of CGRP receptor antagonists in that the active ingredient does not precipitate out upon dilution with saliva. Also, the liquid pharmaceutical compositions of the instant invention have a fast onset of treatment.

### SUMMARY OF THE INVENTION

The instant invention relates to liquid pharmaceutical compositions containing CGRP receptor antagonists. The CGRP receptor antagonist liquid pharmaceutical compositions of the instant invention are alcohol-free, low volume liquid pharmaceutical compositions that can be taken without water for the treatment of migraine headache. Also disclosed are processes for making said pharmaceutical compositions.

### DETAILED DESCRIPTION OF THE INVENTION

The instant invention relates to liquid pharmaceutical compositions containing CGRP receptor antagonists. The CGRP receptor antagonist liquid pharmaceutical compositions of the instant invention comprise a CGRP receptor antagonist, or a pharmaceutically acceptable salt thereof and a pharmaceutically acceptable carrier.

A particularly effective CGRP receptor antagonist is (*S*)-*N*-((3*S*,5*S*,6*R*)-6-methyl-2-oxo-5-phenyl-1-(2,2,2-trifluoroethyl)piperidine-3-yl)-2'-oxo-1',2',5,7-tetrahydrospiro[cyclopenta[*b*]pyridine-6,3'-pyrrolo[2,3-*b*]pyridine]-3-carboxamide trihydrate, 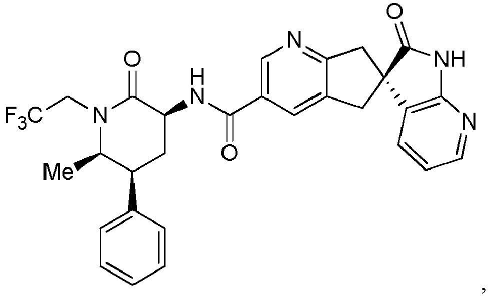 which can be prepared by procedures described in: International Publication WO2012/064910, which published on May 18, 2012, to Merck Sharp & Dohme Corp., will also be referred to as "Compound I" herein. (*S*)-*N*-((3*S*,5*S*,6*R*)-6-methyl-2-oxo-5-phenyl-1-(2,2,2-trifluoroethyl)piperidine-3-yl)-2'-oxo-1',2',5,7-tetrahydrospiro[cyclopenta[*b*]pyridine-6,3'-pyrrolo[2,3-*b*]pyridine]-3-carboxamide is also known by it's generic name, ubrogepant. Compound I is a BCS Class 4 compound, which has low solubility and low permeability (16.6 x10⁻⁶ cm/s). In the liquid pharmaceutical compositions of the instant invention, Compound I stays in solution and does not precipitate immediately upon dilution with saliva.

The liquid pharmaceutical compositions of the present invention may also contain one or more additional formulation ingredients that may be selected from a wide variety of excipients known in the pharmaceutical formulation art. According to the desired properties of the liquid pharmaceutical compositions, any number of ingredients may be selected, alone or in combination, based upon their known uses in preparing liquid pharmaceutical compositions. Such ingredients include, but are not limited to, flavors, flavor enhancers, sweeteners, preservatives and colorants.

The term "liquid pharmaceutical compositions" as used herein is intended to encompass solutions comprising CGRP receptor antagonists.

The pharmaceutical compositions of the present invention are liquid pharmaceutical solutions that comprise a CGRP receptor antagonist and a pharmaceutically acceptable carrier.

The pharmaceutical compositions of the present invention are liquid pharmaceutical solutions that comprise (*S*)-*N*-((3*S*,5*S*,6*R*)-6-methyl-2-oxo-5-phenyl-1-(2,2,2-trifluoroethyl)piperidine-3-yl)-2'-oxo-1',2',5,7-tetrahydrospiro[cyclopenta[*b*]pyridine-6,3'-pyrrolo[2,3-*b*]pyridine]-3-carboxamide or a pharmaceutically acceptable salt thereof and a pharmaceutically acceptable carrier, wherein the volume of the carrier is less than 10 mL. In an embodiment of the invention, the liquid pharmaceutical solutions comprise an amorphous form of (*S*)-*N*-((3S,5S,6*R*)-6-methy1-2-oxo-5-phenyl-1-(2,2,2-trifluoroethyl)piperidine-3-yl)-2'-oxo-1',2',5,7-tetrahydrospiro[cyclopenta[*b*]pyridine-6,3'-pyrrolo[2,3-*b*]pyridine]-3-carboxamide. In another embodiment of the invention, the liquid pharmaceutical solutions comprise an anhydrous form of (*S*)-*N*-((3*S*,5*S*,6*R*)-6-methyl-2-oxo-5-phenyl-1-(2,2,2-trifluoroethyl)piperidine-3-yl)-2'-oxo-1',2',5,7-tetrahydrospiro[cyclopenta[*b*]pyridine-6,3'-pyrrolo[2,3-*b*]pyridine]-3-carboxamide. In another embodiment of the invention, the liquid pharmaceutical solutions comprise a hydrated form of (*S*)-*N*-((3*S*,5*S*,6*R*)-6-methyl-2-oxo-5-phenyl-1-(2,2,2-trifluoroethyl)piperidine-3-yl)-2'-oxo-1',2',5,7-tetrahydrospiro[cyclopenta[*b*]pyridine-6,3'-pyrrolo[2,3-*b*]pyridine]-3-carboxamide. In a class of the invention, the pharmaceutical compositions of the present invention are liquid pharmaceutical solutions that comprise (*S*)-*N*-((3*S*,5*S*,6*R*)-6-methyl-2-oxo-5-phenyl-1-(2,2,2-trifluoroethyl)piperidine-3-yl)-2'-oxo-1',2',5,7-tetrahydrospiro[cyclopenta[*b*]pyridine-6,3'-pyrrolo[2,3-*b*]pyridine]-3-carboxamide trihydrate.

In an embodiment of the invention, the pharmaceutically acceptable carrier comprises a hydrophilic carrier and a water soluble surfactant or mixture of water soluble surfactants.

In an embodiment of the invention, the hydrophilic carrier comprises water, glycols, glycol esters and combinations thereof.

In an embodiment of the invention, glycol is selected from the group consisting of propylene glycol, PEG and glycerol; glycol ester is selected from the group consisting of glycerol esters and propylene glycol esters of organic acids, or mixtures thereof. In a class of the invention, glycol is selected from the group consisting of propylene glycol, PEG-400, glycerol, or mixtures thereof. In a class of the invention, the organic acids have two or three carbon atoms.

In an embodiment of the invention, glycol esters comprise triacetin, triethyl citrate or mixtures thereof.

In an embodiment of the invention, the water soluble surfactant is selected from the group consisting of VitE-TPGS, poloxamer, Tween 20, Tween 80, Span 20, and combinations thereof. In a class of the invention, the surfactant is VitE-TPGS. In a class of the invention, the surfactant is poloxamer. In another class of the invention, the surfactant is poloxamer with Tween 20. In another class of the invention, the surfactant is poloxamer with Tween 80. In another class of the invention, the surfactant is poloxamer with Span 20. In a subclass of the invention, the poloxamer is poloxamer 407. In another class of the invention, the surfactant is VitE-TPGS with Tween 20. In another class of the invention, the surfactant is VitE-TPGS with Tween 80. In another class of the invention, the surfactant is VitE-TPGS with Span 20.

In an embodiment of the invention, the water soluble surfactant is present in an amount of about 0.1% to 15.0% by weight of the composition. In a class of the invention, the water soluble surfactant is present in an amount of 2.5% to 10% by weight of the composition. A suitable water soluble surfactant is VitE-TPGS. Another suitable water soluble surfactant is poloxamer 407.

In an embodiment of the invention, the CGRP receptor antagonist is present in an amount of about 0.01% to 3.0% by weight of the composition. In a class of the invention, the CGRP receptor antagonist is present in an amount of 0.25% to 2.0% by weight of the composition. As an example of the invention, the CGRP Receptor antagonist is (*S*)-*N-*((3*S*,5*S*,6*R*)-6-methyl-2-oxo-5-phenyl-1-(2,2,2-trifluoroethyl)piperidine-3-yl)-2'-oxo-1',2',5,7-tetrahydrospiro[cyclopenta[*b*]pyridine-6,3'-pyrrolo[2,3-*b*]pyridine]-3-carboxamide trihydrate or a salt thereof.

In an embodiment of the invention, the volume of the carrier is less than 5 mL.

In an embodiment of the invention, comprising one or more pharmaceutically acceptable excipients selected from an anti-nucleating polymer, an antioxidant (such as ascorbic acid), a chelating agent (such as ethylenediaminetetraacetic acid (EDTA)), a souring agent, sodium chloride, colors (such as water and organic soluble dyes), sweeteners, flavoring agents or mixtures thereof.

In an embodiment of the invention, the anti-nucleating polymer is selected from the group consisting of Povidone and Kollidone-VA64.

In an embodiment of the invention, the souring agent is selected from citric acid, malic acid, lactic acid, sodium citrate and combinations thereof.

In an embodiment of the invention, the flavoring agent is selected from the group consisting of mint, peppermint, berries, cherries, menthol and sodium chloride flavoring agents, and combinations thereof.

In an embodiment of the invention, the sweetener is selected from the group consisting of sugar, sucralose, aspartame, acesulfame, neotame, and combinations thereof. In a class of the invention, the sweetener is selected from the group consisting of sucralose, aspartame, acesulfame, neotame, and combinations thereof. In a subclass of the invention, the sweetener is sucralose.

Exemplifying the invention is a liquid pharmaceutical composition comprising (*S*)-*N*-((3*S*,5*S*,6*R*)-6-methyl-2-oxo-5-phenyl-1-(2,2,2-trifluoroethyl)piperidine-3-yl)-2'-oxo-1',2',5,7-tetrahydrospiro[cyclopenta[*b*]pyridine-6,3'-pyrrolo[2,3-*b*]pyridine]-3-carboxamide, propylene glycol, PEG-400, Water, VitE-TPGS, Povidone, Sucralose, Menthol, and a peppermint flavor. Further exemplifying the invention is a liquid pharmaceutical composition comprising (*S*)-*N*-((3*S*,5*S*,6*R*)-6-methyl-2-oxo-5-phenyl-1-(2,2,2-trifluoroethyl)piperidine-3-yl)-2'-oxo-1',2',5,7-tetrahydrospiro[cyclopenta[*b*]pyridine-6,3'-pyrrolo[2,3-*b*]pyridine]-3-carboxamide, propylene glycol, PEG-400, Water, VitE-TPGS, Povidone, Sucralose, Menthol, a peppermint or mint flavor, a souring agent and sodium chloride.

The liquid pharmaceutical compositions of the instant invention are stable at room temperature. Specifically, the liquid pharmaceutical compositions have good physical and chemical stability at room temperature, in a range of about 25° C (±2° C) to 40° C (±2° C).

The liquid pharmaceutical compositions of the instant invention have a low viscosity (∼ 0.065 Pa.s) at room temperature. It is desirable to have a liquid pharmaceutical composition with low viscosity that can exit the container quickly. In an embodiment of the invention, the liquid pharmaceutical composition exits the container within 1-10 seconds. In a class of the invention, the liquid pharmaceutical composition exits the container within 1-5 seconds.

The liquid pharmaceutical compositions of the instant invention comprise a level of excipients that are suitable for daily application and the allowance for a single re-administration as needed.

The liquid pharmaceutical compositions of the instant invention are suitable for packaging into multi-dose or unit-dose packages without producing discoloration or degradation.

The instant invention includes a process of preparing a liquid pharmaceutical composition of (*S*)-*N*-((3*S*,5*S*,6*R*)-6-methyl-2-oxo-5-phenyl-1-(2,2,2-trifluoroethyl)piperidine-3-yl)-2'-oxo-1',2',5,7-tetrahydrospiro[cyclopenta[*b*]pyridine-6,3'-pyrrolo[2,3-*b*]pyridine]-3-carboxamide trihydrate comprising the steps of:
(i) Dissolving the water soluble surfactant in a hydrophilic carrier at room temperature, or at higher temperatures (about 40°C), until a homogenous solution is formed by continuous stirring;
(ii) Dissolving additional excipients, such as a flavoring agent, in solution from Step (i);
(iii) Dissolving water soluble excipients (such as an anti-nucleating polymer, sweeteners, flavoring agents) in water;
(iv) Mixing the solution from step (iii) with the solution of step (ii) by continuous stirring;
(v) Dissolving (*S*)-*N*-((3*S*,5*S*,6*R*)-6-methyl-2-oxo-5-phenyl-1-(2,2,2-trifluoroethyl)piperidine-3-yl)-2'-oxo-1',2',5,7-tetrahydrospiro[cyclopenta[*b*]pyridine-6,3'-pyrrolo[2,3-*b*]pyridine]-3-carboxamide thrihydrate in the solution of step (iv) by continuous stirring at room temperature or 40°C until a homogenous solution is formed;
(vi) Dissolving one or more excipients in the solution from step (v).

The liquid pharmaceutical compositions of the present invention may be useful in the therapeutic or prophylactic treatment of disorders associated with CGRP functioning. Such disorders include: migraine and cluster headache; chronic tension type headache; chronic pain; neurogenic inflammation and inflammatory pain; eye pain; tooth pain; non-insulin dependent diabetes mellitus; vascular disorders; inflammation; arthritis; bronchial hyperreactivity; asthma; shock; sepsis; opiate withdrawal syndrome; morphine tolerance; hot flashes in men and women; allergic dermatitis; psoriasis; encephalitis, brain trauma, ischaemia, stroke, epilepsy, and neurodegenerative diseases; skin diseases; neurogenic cutaneous redness, skin rosaceousness and erythema; tinnitus; obesity; inflammatory bowel disease; irritable bowel syndrome; and cystitis. In a class of the invention, the liquid pharmaceutical compositions of the instant invention may be useful in the acute treatment or prophylactic treatment of headache, including migraine and cluster headache.

The following examples are given for the purpose of illustrating the present invention and shall not be construed as being limitations on the scope of the invention.

### EXAMPLE 1

### ORAL LIQUID FORMULATIONS WITH (S)-N-((3S,5S,6R)-6-METHYL-2-OXO-5-PHENYL-1-(2,2,2-TRIFLUOROETHYL)PIPERIDINE-3-YL)-2'-OXO-1',2',5,7-TETRAHYDROSPIRO[CYCLOPENTA[B]PYRIDINE-6,3'-PYRROLO[2,3-B]PYRIDINE]-3-CARBOXAMIDE TRIHYDRATE (COMPOUND I)

| Ingredient | A | B | C | D | E | F | G |
|---|---|---|---|---|---|---|---|
| | 50mg/4g or /3.7mL | 25mg/4g or /3.7mL | 50 mg/4g or /3.7mL | 50mg/3g or /2.78 mL | 50mg /3.24g or /3mL | 50 mg /3.24g or /3mL | 50 mg /3.24g or /3mL |
| Compound I | 1.38 | 0.69 | 1.38 | 1.83 | 1.694 | 1.694 | 1.694 |
| PEG 400 (USP/NF, Ph. Eur, JP) | 33.61 | 33.96 | 33.72 | 33.27 | 33.406 | 39.256 | 34.256 |
| Propylene Glycol (USP, EP) | 33.61 | 33.96 | 33.5 | 33.5 | 33.5 | 36 | 31 |
| VitE- TPGS (NF) | 10.00 | 10.00 | 10.00 | 10.00 | 10.00 | 0.00 | 10.00 |
| PVP (USP, EP) | 1.00 | 1.00 | 1.00 | 1.00 | 1.00 | 1.00 | 1.00 |
| Water (USP) | 20.00 | 20.00 | 20.00 | 20.00 | 20.00 | 20.00 | 20.00 |
| Sucralose | 0.10 | 0.10 | 0.10 | 0.10 | 0.10 | 0.50 | 0.50 |
| Souring agents | | | | | | 0.50 | 0.50 |
| Sodium Chloride | | | | | | 0.50 | 0.50 |
| Flavor agent | 0.30 | 0.30 | 0.30 | 0.30 | 0.30 | 0.50 | 0.50 |
| Menthol | | | | | | 0.05 | 0.05 |
| Total (g) | 100.00 | 100.01 | 100.00 | 100.00 | 100.00 | 100.00 | 100.00 |
| Total (mL) | 92.59 | 92.60 | 92.59 | 92.59 | 92.59 | 92.59 | 92.59 |

The oral liquid formulations provided above are prepared as follows:
1) Add VitE-TPGS (as small pieces <4 mm) to Propylene Glycol and warm up the mixture to about 40°C in a glass bottle (Use a stirrer bar and a magnet stirrer / heater).
2) Stir until VitE-TPGS dissolves completely and provides a clear solution (∼ <1 h).
3) Turn-off the heat.
4) Add PEG-400 to "VitE-TPGS- Propylene Glycol" solution and stir to mix (the solution will be somewhat hazy) (∼ 5 min).
5) Dissolve additional excipients, such as Menthol, in solution from Step (4).
6) Add PVP to "Water" in a beaker and stir to dissolve (∼<30 min).
7) Add sweetener, salt, souring agent and stir to dissolve (∼10 min).
8) Add "PVP-sweetener- salt- souring agent solution" to "VitE-TPGS- PG- PEG-400 solution" and stir to mix (∼ <10 min).
9) Add Compound I to the above solution (Step 8) and stir to dissolve (warm up the solution to about ∼ 40°C for faster dissolution) (∼10 min). Solutions should be clear.
10) Add flavor agent and stir to mix (∼5 min).

### EXAMPLE 2

### PHARMACOKINETICS OF (S)-N-((3S,5S,6R)-6-METHYL-2-OXO-5-PEENYL-1-(2,2,2-TRIFLUOROETHYL)PIPERIDINE-3-YL)-2'-OXO-1',2',5,7-TETRAHYDROSPIRO[CYCLOPENTA[B]PYRIDINE-6,3'-PYRROLO[2,3-B]PYRIDINE]-3-CARBOXAMIDE TRIHYDRATE (COMPOUND I) ORAL LIQUID FORMULATIONS IN DOGS

In order to evaluate the effect of solution volume on exposure, two formulations were tested in dogs; each solution had the same dose of Compound I, but the volume of solution differed. The oral solution formulations were delivered to the dogs via syringe to the back of the throat (to simulate swallowing of a small volume liquid solution). No rinse was given with the oral solution formulations. For the solutions, 0.75 mL of high and 1.5mL of low drug concentration solutions were used. For the PEG reference solution, 10 mg dose is given in 20 mL vehicle followed by 15 mL of water.

### PK of Compound I in dog from low volume oral PVE solution in fasted, pentagastrin pretreated beagle dogs

| Dosing Formulations | N | Dose (mg) | AUC₀₋₂₄ₕᵣ (µM*hr) | nAUC_{0- 24hr} (µM*hr] | C ₘₐₓ (µM) | AUC₀₋₂ₕᵣ (nM*hr) | C₂ₕ (µM] | Tₘₐₓ* (hr) |
|---|---|---|---|---|---|---|---|---|
| High concentration oral solution | 6 | 9.33 | 0.685 ± 0.044 | 0.073 ± 0.004 | 0.454 + 0.025 | 0.479 ± 0.03 | 0.119 ± 0.012 | 0.5 |
| Low concentration oral solution | 6 | 10 | 0.878 ± 0.146 | 0.087 ± 0.014 | 0.520 ± 0.036 | 0.580 ± 0.069 | 0.154 ±0.035 | 0.5 (0.5-1) |
| *PEG 400 solution*** | *6* | *10* | *0.534 ± 0.068* | *0.053 ± 0.006* | *0.263 ± 0.042* | *0.364 ± 0.054* | *0.115 ± 0.013* | *0.5 (0.5-1)* |
| *For Tmax, median value is provided, ** Data from different dogs | | | | | | | | |

In dogs, both oral solutions have slightly better exposure than the previously generated PEG 400 solution data.

### Oral Solutions

| Ingredient | High concentration oral solution | Low concentration oral solution |
|---|---|---|
| | 13.34 mg/mL | 6.67 mg / mL |
| | % W/W | % W/W |
| Compound I | 1.356 | 0.678 |
| PEG 400 (USP/NF, Ph. Eur, JP) | 33.772 | 34.111 |
| Propylene Glycol (USP, EP) | 33.772 | 34.111 |
| VitE- TPGS (NF) | 10.000 | 10.000 |
| PVP (USP, EP) | 1.000 | 1.000 |
| Sucralose | 0.100 | 0.100 |
| Water | 20.000 | 20.000 |
| Total (g) | 100.000 | 100.000 |

PEG 400 solution: PEG 400/Orasweet/Water 70%/15%/15% (10 mg dose in 20 mL vehicle followed by 15 mL of water). The PEG 400 solution is not suitable for chronic use due to high amount of PEG400. It is only suitable as a single dose in early clinical studies.

### EXAMPLE 3

### PRECIPITATION OF (S)-N-((3S,5S,6R)-6-METHYL-2-OXO-5-PHENYL-1-(2,2,2-TRIFLUOROETHYL)PIPERIDINE-3-YL)-2'-OXO-1',2',5,7-TETRAHYDROSPIRO[CYCLOPENTA[B]PYRIDINE-6,3'-PYRROLO[2,3-B]PYRIDINE]-3-CARBOXAMIDE TRIHYDRATE (COMPOUND I) IN ARTIFICIAL SALIVA UPON DILUTION

Samples of Compound I oral liquid formulation were diluted in 1:1 or 1:4 ratios with pH 6.2 artificial saliva. Artificial saliva comprises: KH₂PO₄ (12mM), NaCl (40 mM), CaCl₂ (1.5 mM), and NaOH to pH 6.2 (Reference: Ritschel and Thomson, Methods and findings in Experimental and Clinical Pharmacology, %, 511-525, 1983). Magnetic stirring at 400RPM was used. Samples were taken at various time points to determine the potential precipitation kinetics upon dilution. Centrifuge filtration with 0.45µm filter was used for the 5 minute time point, and 1µm filtration and ultracentrifugation (80k RPM, 25°C, 15minutes, 9/9 acceleration/deceleration) were used for longer time points. Samples were diluted with 50/50 ACN/water to try to match analytical standard concentration (0.1mg/mL) and analyzed with HPLC. The LC conditions used are as follows: 65% 0.1%H3PO4/35%ACN isocratic method, Chromolith SpeedROD RP-18e 50x4.6mm column, UV at 210nm, 40°C column temperature, 3mL/min flow rate, and 10uL injection volume.

Precipitation Summary: Drug concentration of the formulation A (Described in EXAMPLE 1) after dilution 1:1 and 1:4 with saliva pH 6.2

| Dilution | Formulation | Drug Concentration (mg/mL) | *Time (minutes)* | mg/mL | Separation Technique | %released |
|---|---|---|---|---|---|---|
| 1:1 | A | 12.5 | *5* | 4.91379 | 0.45um syringe filtered | 79% |
| | | | *30* | 5.41665 | 0.45um syringe filtered | 87% |
| | | | *120* | 5.50393 | 0.45um syringe filtered | 88% |
| 1:4 | A | 12.5 | *5* | 2.42852 | 0.45um syringe filtered | 97% |
| | | | *30* | 2.43728 | 0.45um syringe filtered | 97% |
| | | | *120* | 0.54545 | 0.45um syringe filtered | 22% |

| | | | | | | |
|---|---|---|---|---|---|---|
| When solution A was diluted 1:1, Compound I did not precipitate, and the concentration remained at the expected level (almost ½ of original concentration). When solution A was diluted 1:4, the drug concentration remained at the expected level (about 1/5 of the original concentration) for about 30 minutes, and then slowly started to precipitate out. These results indicate that solution A resists precipitation upon dilution in the mouth with saliva. | | | | | | |

### EXAMPLE 4

### VISCOSITY OF (S)-N-((3S,5S,6R)-6-METHYL-2-OXO-5-PHENYL-1-(2,2,2-TRIFLUOROETHYL)PIPERIDINE-3-YL)-2'-OXO-1',2',5,7-TETRAHYDROSPIRO[CYCLOPENTA[B]PYRIDINE-6,3'-PYRROLO[2,3-B]PYRIDINE]-3-CARBOXAMIDE TRIHYDRATE (COMPOUND I) ORAL LIQUID FORMULATIONS

To measure viscosity, TA Instruments ARES G2 strain controlled rheometer with TRIOS software was used. A double wall configuration was used due to low viscosity of the sample. All liquids appear to be Newtonian between 5-20 s⁻¹. 5 minutes was selected for isothermal holding time, which was considered reaching equivalence. As shown below, the three formulations tested all demonstrated low viscosity (<0.065).

| Ingredient | A | BJ | CJ |
|---|---|---|---|
| | 50 mg/4 g or /3.7 mL | | |
| | % WAV | %w/w | % WAV |
| Ethanol | | 5.00 | |
| PEG 400 (USP/NF, Ph.Eur, JP) | 33.61 | 31.11 | 36.11 |
| Propylene Glycol | 33.61 | 31.11 | 36.11 |
| VitE- TPGS (NF) | 10.00 | 10.00 | 5.00 |
| PVP USP, EP) | 1.00 | 1.00 | 1.00 |
| Compound I | 1.38 | 1.38 | 1.38 |
| Sucralose | 0.10 | 0.10 | 0.10 |
| Flavor | 0.30 | 0.30 | 0.30 |
| Water | 20.00 | 20.00 | 20.00 |
| Total | 100 | 100 | 100 |

Viscosity of liquid formulations at 25°C and 10°C.

| Sample | Viscosity (η, Pa·s) | |
|---|---|---|
| | 25°C | 10°C |
| A Placebo | 0.065 | 0.143 |
| A | N/D* | N/D* |
| BJ | 0.053 | 0.114 |
| BJ Placebo | 0.05 | 0.108 |
| CJ | 0.03 | 0.062 |
| CJ Placebo | 0.041 | 0.088 |

| | | |
|---|---|---|
| *The value should be similar to the placebo viscosity | | |

Preferred Embodiments:
1. A liquid pharmaceutical composition comprising (*S*)-*N*-((3*S*,5*S*,6*R*)-6-methyl-2-oxo-5-phenyl-1-(2,2,2-trifluoroethyl)piperidine-3-yl)-2'-oxo-1',2',5,7-tetrahydrospiro[cyclopenta[*b*]pyridine-6,3'-pyrrolo[2,3-*b*]pyridine]-3-carboxamide, or a pharmaceutically acceptable salt thereof, and a pharmaceutically acceptable carrier, wherein the volume of the carrier is less than 10 mL.
2. The liquid pharmaceutical composition according to Embodiment 1 comprising (*S*)-*N*-((3*S*,5*S*,6*R*)-6-methyl-2-oxo-5-phenyl-1-(2,2,2-trifluoroethyl)piperidine-3-yl)-2'-oxo-1',2',5,7-tetrahydrospiro[cyclopenta[*b*]pyridine-6,3'-pyrrolo[2,3-*b*]pyridine]-3-carboxamide trihydrate.
3. The liquid pharmaceutical composition according to Embodiment 1, wherein the carrier comprises a hydrophilic carrier and a water soluble surfactant, or mixture of water soluble surfactants.
4. The liquid pharmaceutical composition according to Embodiment 3, wherein the hydrophilic carrier comprises water, glycols, glycol esters or a combination thereof.
5. The liquid pharmaceutical composition according to Embodiment 4, comprising glycols selected from the group consisting of propylene glycol and PEG; and glycol ester selected from the group consisting of glycerol esters, propylene glycol esters of organic acids or mixtures thereof.
6. The liquid pharmaceutical composition according to Embodiment 5, wherein glycol is selected from the group consisting of propylene glycol, PEG-400, glycerol and mixtures thereof.
7. The liquid pharmaceutical composition according to Embodiment 5, wherein glycol ester is selected from the group consisting of triacetin, triethyl citrate and mixtures thereof.
8. The liquid pharmaceutical composition according to Embodiment 3, wherein the water soluble surfactant is selected from the group consisting of VitE-TPGS, Poloxamer, Tween 20, Tween 80 and Span 20, and combinations thereof.
9. The liquid pharmaceutical composition according to Embodiment 8 wherein the water soluble surfactant is selected from the group consisting of VitE-TPGS, poloxamer, poloxamer with Tween 20, poloxamer with Tween 80, poloxamer with Span 20, VitE-TPGS with Tween 20, VitE-TPGS with Tween 80 and VitE-TPGS with Span 20.
10. The liquid pharmaceutical composition according to Embodiment 3, wherein the water soluble surfactant is present in an amount of about 0.1% to 15.0% by weight of the composition.
11. The liquid pharmaceutical composition according to Embodiment 10, wherein the water soluble surfactant is present in an amount of 2.5% to 10% by weight of the composition.
12. The liquid pharmaceutical composition according to Embodiment 11 wherein the water soluble surfactant is VitE-TPGS or poloxamer.
13. The liquid pharmaceutical composition according to Embodiment 1, wherein (*S*)-*N*-((3*S*,5*S*,6*R*)-6-methyl-2-oxo-5-phenyl-1-(2,2,2-trifluoroethyl)piperidine-3-yl)-2'-oxo-1',2',5,7-tetrahydrospiro[cyclopenta[*b*]pyridine-6,3'-pyrrolo[2,3-*b*]pyridine]-3-carboxamide or a salt thereof is present in an amount of about 0.01% to 3.0% by weight of the composition.
14. The liquid pharmaceutical composition according to Embodiment 13, wherein (*S*)-*N*-((3*S*,5*S*,6*R*)-6-methyl-2-oxo-5-phenyl-1-(2,2,2-trifluoroethyl)piperidine-3-yl)-2'-oxo-1',2',5,7-tetrahydrospiro[cyclopenta[*b*]pyridine-6,3'-pyrrolo[2,3-*b*]pyridine]-3-carboxamide or a salt thereof is present in an amount of 0.25% to 2.0% by weight of the composition.
15. The liquid pharmaceutical composition according to Embodiment 1, wherein the volume of carrier is less than 5 mL.
16. The liquid pharmaceutical composition according to Embodiment 1, further comprising one or more pharmaceutically acceptable excipients selected from the group consisting of anti-nucleating polymers, antioxidants, chelating agents, souring agents, sodium chloride, colors, sweeteners and flavors.
17. The liquid pharmaceutical composition according to Embodiment 1 comprising propylene glycol, PEG-400, Water, VitE-TPGS, Povidone, Sucralose, Menthol, and a mint or peppermint flavor.
18. The liquid pharmaceutical composition according to Embodiment 17, which further comprises a souring agent.
19. A method of treating migraine headache by administering a liquid pharmaceutical composition according to Embodiment 1.
20. A method of treating migraine headache by administering a liquid pharmaceutical composition according to Embodiment 17.

## Claims

1. A liquid pharmaceutical composition comprising (*S*)-*N*-((3*S*,5S, 6*R*)-6-methy1-2-oxo-5-phenyl-1-(2,2,2-trifluoroethyl)piperidine-3-yl)-2'-oxo-1',2',5,7-tetrahydrospiro[cyclopenta[*b*]pyridine-6,3'-pyrrolo[2,3-*b*]pyridine]-3-carboxamide trihydrate and a pharmaceutically acceptable carrier comprising VitE-TPGS.

2. The liquid pharmaceutical composition according to Claim 1, wherein the carrier further comprises one or more of water, a glycol, and a glycol ester.

3. The liquid pharmaceutical composition according to Claim 2, wherein the glycol is selected from propylene glycol, PEG, glycerol, and mixtures thereof; and the glycol ester is selected from glycerol esters, propylene glycol esters of organic acids, and mixtures thereof.

4. The liquid pharmaceutical composition according to Claim 3, wherein the glycol is selected from propylene glycol, PEG-400, glycerol, and mixtures thereof.

5. The liquid pharmaceutical composition according to Claim 3, wherein the glycol ester is selected from triacetin, triethyl citrate, and mixtures thereof.

6. The liquid pharmaceutical composition according to Claim 1, wherein the VitE-TPGS is present in an amount of 0.1% to 15.0% by weight of the composition.

7. The liquid pharmaceutical composition according to Claim 6, wherein the VitE-TPGS is present in an amount of 2.5% to 10% by weight of the composition

8. The liquid pharmaceutical composition according to Claim 1, wherein the volume of the carrier is less than 5 mL.

9. The liquid pharmaceutical composition according to Claim 1, further comprising one or more pharmaceutically acceptable excipients selected from anti-nucleating polymers, antioxidants, chelating agents, souring agents, sodium chloride, colors, sweeteners and flavors.

10. The liquid pharmaceutical composition according to Claim 1, comprising propylene glycol, PEG-400, water, povidone, sucralose, menthol, and a mint or peppermint flavor.

11. The liquid pharmaceutical composition according to Claim 10, further comprising a souring agent.

12. A liquid pharmaceutical composition according to Claim 1 for use in a method of treating migraine headache.

13. A liquid pharmaceutical composition according to Claim 10 for use in a method of treating migraine headache.
